# EUROPEAN PATENT APPLICATION

(11) **EP 3 868 290 A1**
(43) Date of publication of application: **25.08.2021**
(21) Application number: 19873931.0
(22) Date of filing: 16.10.2019
(51) Int. Cl.: A61B 5/0478

(54) **BIOELECTRODE AND BIOELECTRODE PRODUCTION METHOD**

(30) Priority: 17.10.2018 JP 2018195921; 17.10.2018 JP 2018195922
(71) Applicant: NOK Corporation, Minato-ku Tokyo 105-8585 (JP)
(72) Inventor: YOSHITOMI Takumi, Tsujido-shinmachi, Fujisawa-shi, Kanagawa 251- 0042 (JP); FUTASHIMA Ryo, Tsujido-shinmachi, Fujisawa-shi, Kanagawa 251- 0042 (JP); UDA Toru, Tokyo 105-0004 (JP)
(74) Representative: TBK
(86) International application number: PCT/JP2019/040640
(87) International publication number: WO 2020/080395

(57) **Abstract**

Provided is a bioelectrode having a structure not complicated, having satisfactory elasticity, and capable of reducing contact impedance.

A bioelectrode (1) includes a support member (2), which is a conductive member, and at least one electrode member (3), which is a member projecting from the support member (2). The at least one electrode member (3) is molded from a conductive rubber containing a silicone rubber and metal particles, and the electrode member (3) has an exposed face (32), which is a face formed by removal of a molded surface layer at a tip end (31).

## Description

### Technical Field

The present invention relates to a bioelectrode and a method for producing a bioelectrode, and specifically relates to a bioelectrode for detecting biosignals such as brainwaves and a method for producing the bioelectrode.

### Background Art

Bioelectrodes have been conventionally used for detecting biosignals. Bioelectrodes are used while being placed in contact with a subject's body. Bioelectrodes are used, for example, in order to detect brainwave signals for analysis of a brain function state with a purpose of early detecting Alzheimer's disease and the like. A bioelectrode for detecting brainwaves is used while its electrode member is directly placed in contact with the scalp of a subject in order to detect brainwave signals. Conventional bioelectrodes include those in the form of a plate-like thin sheet made of a highly conductive metal such as silver or gold. These thin sheet bioelectrodes have insufficient adhesion with skin. In order to lower contact impedance between the electrode and skin, it has been necessary to apply a gel, cream, paste, or the like between the skin and the bioelectrode. These substances applied have to be removed after detection of biosignals, and use thereof has required an effort. Additionally, an electric double layer is formed by ionization of the metal on the interface between the skin and the electrode to thereby generate a polarization voltage. The variation in this polarization voltage causes a baseline variation. In order to stabilize the polarization voltage, there has been required aging for forming a silver chloride film on an electrode surface for a silver electrode.

In contrast, as bioelectrodes requiring no application of a gel or the like, there are bioelectrodes including a metal probe (e.g., see Patent Literature 1) and bioelectrodes formed by impregnating a water-absorbent member such as a sponge with an electrolyte solution in which an amino acid or organic salt dissolved (e.g., see Patent Literature 2).

### Document List

### Patent Literatures

Patent Literature 1: Japanese Patent Application Publication No. 2013-248306
Patent Literature 2: Japanese Patent Application Publication No. 2013-144051

### Summary of Invention

### Technical Problem

Metal probes, which are rigid, may cause pain to a subject when allowed to adhere to the scalp. In the case where a plurality of metal probes is provided, a measure is required to make adhesion of all the probes to the scalp satisfactory. For this reason, as in Patent Literature 1, it is necessary to provide the metal probes with a buffering member such as a spring, and thus, the structure of the probes is complicated.

Alternatively, in the case of a bioelectrode in which an electrolyte solution is used to a water-absorbent member as in Patent Literature 2, its performance is degraded due to drying, wetness leads to discomfort, and so on. Furthermore, it is difficult to form the tip end portion of such an electrode such that to the tip end have a pointed shape, and it is not possible to achieve satisfactory elasticity because the electrode is excessively soft. For this reason, it is necessary to reinforce the electrode with another member in order to allow the electrode to adhere to a living body.

As mentioned above, a configuration in which the structure is not complicated, satisfactory elasticity is provided so as not to cause discomfort to a subject, and contact impedance can be reduced has been required from conventional bioelectrodes.

The present invention has been made in view of the above-described problems, and it is an object of the present invention to provide a bioelectrode having a structure not complicated, having a satisfactory elasticity, and capable of reducing contact impedance, and a method for producing a bioelectrode.

### Solution to Problem

In order to achieve the object described above, a bioelectrode according to the present invention is characterized by comprising a support member, which is a conductive member, and at least one electrode member, which is a member projecting from the support member, wherein at least the electrode member is molded from a conductive rubber containing a silicone rubber and metal particles, and the electrode member has an exposed face, which is a face formed by removal of a molded surface layer at the tip end portion of the electrode member.

In the bioelectrode according to one aspect of the present invention, the exposed face is formed inclinedly with respect to a support face that supports the electrode member in the support member, and the tip end portion is pointed.

In the bioelectrode according to one aspect of the present invention, the inclination of each exposed face is the same with respect to the support face.

In the bioelectrode according to one aspect of the present invention, the exposed face is a cut face.

In the bioelectrode according to one aspect of the present invention, the exposed face is a polished face.

In the bioelectrode according to one aspect of the present invention, the exposed face is a flat face.

In the bioelectrode according to one aspect of the present invention, the silicone rubber is a room temperature-curable liquid silicone rubber.

In the bioelectrode according to one aspect of the present invention, the metal particles are silver particles.

In order to achieve the object described above, a method for producing a bioelectrode according to the present invention is a method for producing a bioelectrode comprising a support member, which is a conductive member, and at least one electrode member, which is a member projecting from the support member, the method being characterized by comprising an electrode member molding step of molding the electrode member by stirring a conductive rubber containing a silicone rubber and metal particles and molding the conductive rubber into a shape projecting from the support member, and an exposed face forming step of forming an exposed face, which is a face at which an interior of a molded surface layer is exposed, by removing the molded surface layer, which is a portion of the surface, at a tip end portion of the electrode member molded by the electrode member molding step.

In the method for producing a bioelectrode according to one aspect of the present invention, in the exposed face forming step_{"} the exposed face is formed inclinedly with respect to a support face that supports the electrode member in the support member, and the tip end portion is formed in a manner such that the tip end portion is pointed.

### Effects of Invention

According to the bioelectrode and the method for producing a bioelectrode according to the present invention, the structure is not complicated, satisfactory elasticity is provided, and the contact impedance can be reduced.

### Brief Description of Drawings

[Fig. 1] A perspective view schematically illustrating a configuration of a bioelectrode according to a first embodiment of the present invention.
[Fig. 2] A perspective view from another direction schematically illustrating the configuration of the bioelectrode illustrated in Fig. 1.
[Fig. 3] A front view of an electrode member of the bioelectrode according to the first embodiment of present invention.
[Fig. 4] A front view of an electrode member in an intermediate product molded in an electrode member molding step of a method for producing the bioelectrode according to the first embodiment of present invention.
[Fig. 5] A view for illustrating a contact impedance evaluation test of the bioelectrode according to the first embodiment of present invention.
[Fig. 6] A view showing test results of the contact impedance evaluation test illustrated using Fig. 5.
[Fig. 7] A perspective view schematically illustrating a configuration of a bioelectrode according to a second embodiment of the present invention.
[Fig. 8] A perspective view from another direction schematically illustrating the configuration of the bioelectrode illustrated in Fig. 7.
[Fig. 9] A front view of an electrode member of the bioelectrode according to the second embodiment of present invention.
[Fig. 10] A side view of the electrode member of the bioelectrode according to the second embodiment of present invention.
[Fig. 11] A side view of an electrode member in an intermediate product molded in an electrode member molding step of a method for producing the bioelectrode according to the second embodiment of present invention.
[Fig. 12] A view for illustrating a contact impedance evaluation test of the bioelectrode according to the second embodiment of present invention.
[Fig. 13] A view showing test results of the contact impedance evaluation test illustrated using Fig. 12.

### Description of Embodiments

Hereinafter, embodiments of the present invention will be described with reference to the drawings.

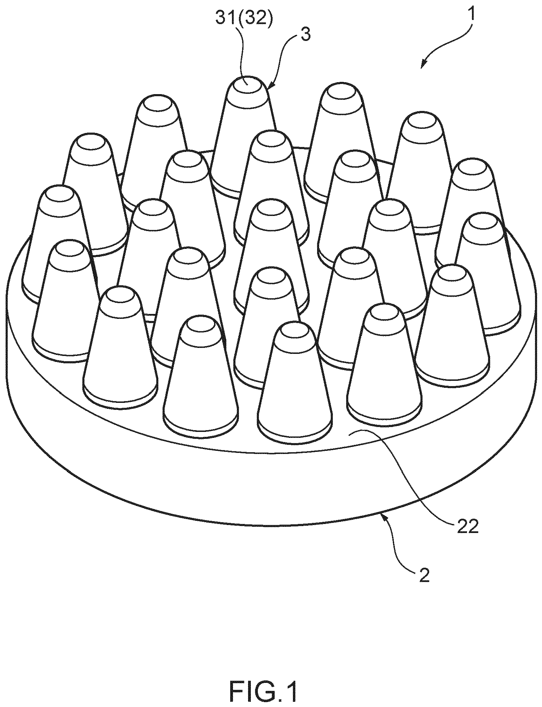

Fig. 1 is a perspective view schematically illustrating a configuration of a bioelectrode according to a first embodiment of the present invention. As shown in Fig. 1, a bioelectrode 1 includes a support member 2, which is a conductive member, and at least one electrode member 3, which is a member projecting from the support member 2. At least the electrode member 3 is molded from a conductive rubber containing a silicone rubber and metal particles, and the electrode member 3 has an exposed face 32, which is a face formed by removal of a molded surface layer at a tip end (tip end 31). The tip end 31 of the electrode member 3 contacts a body of a subject, and the bioelectrode 1 makes biosignals of the subject detectable via the electrode member 3. The bioelectrode 1 is, for example, a bioelectrode for detecting brainwaves which contacts a head of a subject to detect brainwaves. The bioelectrode 1 is not limited to these for detecting brainwaves, and is applicable to other devices for detecting biosignals such as wearable information devices. Hereinafter, the configuration of the bioelectrode 1 will be specifically described.

The support member 2 supports the electrode member 3 and is formed of a conductive material so as to be electrically connected to the electrode member 3. In the bioelectrode 1 according to the present embodiment, the support member 2 and the electrode member 3 are integrally molded, and the support member 2 is also formed of the same conductive rubber as that for the electrode member 3. The shape of the support member 2 may be, as mentioned below, any shape capable of supporting the electrode member 3 in a state where the electrode member 3 is projecting, and is not limited to a specific shape. The support member 2 has a disk shape or substantially disk shape, as shown in Fig. 1, for example.

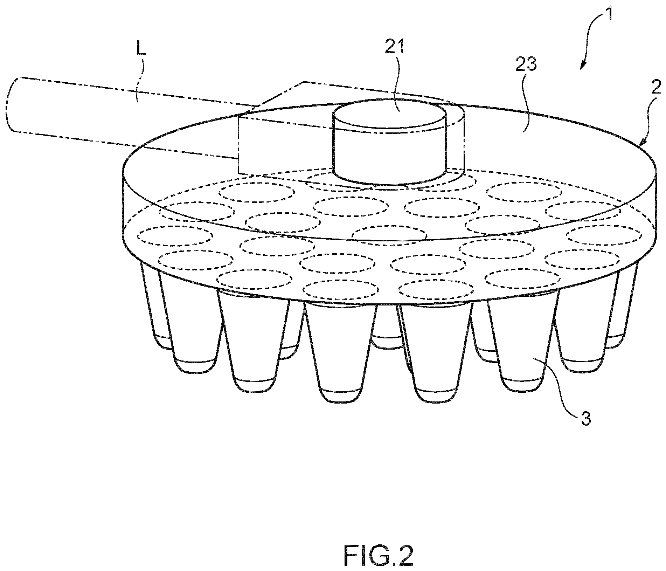

The support member 2 is provided with a terminal 21, which is to be electrically connected to a measurement apparatus, not shown, for receiving biosignals detected by the bioelectrode 1 and processing, analyzing, displaying or the like the biosignals received. The terminal 21 is, for example, as shown in Fig. 2, is connected to a connecting cable L that enables the measurement apparatus to be electrically connected to external devices. The terminal 21 is provided, for example, on a face (terminal side face 23) opposite to the face (support face 22) on which the electrode member 3 is supported, and has a shape to which the connecting cable L can be connected. The terminal 21 is, for example, a projecting portion that projects from the terminal side face 23. In the support member 2, the terminal 21 is integrally formed with other portions by the same conductive rubber, but the terminal may be one formed as a separate body from the other portions by a different material. In such a case, the terminal 21 can be formed of a material suitable for electrical connection to the connecting cable L. For example, the terminal 21 may be produced from a metal, and may be adhesively provided on the terminal side face 23 of the support member 2 by using a conductive adhesive or may be provided by being embedded more internally than the terminal side face 23 such that a portion of the terminal 21 projects from the terminal side face 23.

A plurality of the electrode members 3 is provided in the bioelectrode 1 as shown in Fig. 1, projecting from the support face 22 of the support member 2 in the same or the substantially same direction. The electrode members 3 extend from the support member 2 in a manner such that the tip ends 31 are each located on a same flat face, as shown in Fig. 1. The electrode members 3 project from the support member 2, for example, like a brush, as shown in Fig. 1. The electrode members 3 each may extend from the support member 2 to positions adapted to a target site of a subject such that each of the tip ends 31 contact the target site of the subject in an equivalent state in a usage state. In other words, the tip ends 31 of the electrode members 3 may not be located on a same flat face. The shape of each of the electrode member 3, as shown in Fig. 1, is, for example, a cylindrical or substantially cylindrical shape, and is a shape having a portion tapering towards the tip end 31. Each of the electrode member 3 may have a conical or substantially conical shape tapering toward the tip end 31 entirely, and each of the electrode members 3 is required to have a shape having the tip end 31 and projecting from the support member 2.

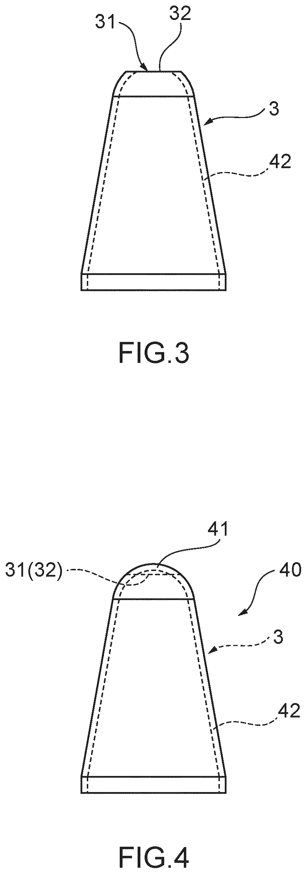

The tip end 31 of each of the electrode members 3 has the exposed face 32, which is a face formed by removal of a molded surface layer, as shown in Figs. 1 and 3. The exposed face 32 is a face facing in a direction in which the electrode member 3 extends, for example, as shown in Fig. 3, is a flat face or substantially flat face, and a face orthogonal or substantially orthogonal to the direction in which the electrode member 3 extends. The exposed face 32 may be a curved face or may be a face having a curved face and a flat face. The exposed face 32 is, as mentioned below, a face formed by removing a molded surface layer 42 of a tip end part 41 from an intermediate product 40 of each electrode member 3 molded from a conductive rubber (see Fig. 4), and a cut face or polished face formed respectively by cutting or polishing tip end part 41 of the intermediate product 40 of each electrode member 3.

The conductive rubber forming the electrode members 3 is a rubber containing a silicone rubber and metal particles, as mentioned above. The silicone rubber is, for example, a room temperature-curable liquid silicone rubber, and the metal particles are, for example, silver particles. The metal particles may be any metal particles as long as being a metal-based material having electrical conductivity. The metal particles may be, for example, particles made of a carbon-based material such as carbon black or carbon nanotubes.

A room temperature-curable liquid silicone rubber is a silicone rubber which is in the form of liquid or paste before curing and turns into a rubber elastic body usually at 20°C to 100°C due to progress of a curing reaction. The curing reactions include a reaction that gradually progresses by moisture (water) in air and a reaction that readily progresses by addition of a curing agent to a main material. The silicone rubber may be one to be cured by either type of the curing reactions. As the room temperature-curable liquid silicone rubber, only one type of room temperature-curable liquid silicone rubber may be used, or a plurality types of room temperature-curable liquid silicone rubbers may be mixed and used.

As silver particles in the conductive rubber, silver particles in the form of particles containing an aggregated silver powder and a flaky silver powder can be used. The aggregated silver powder is a silver powder of a plurality of particulate primary particles three-dimensionally aggregated, and the flaky silver powder a silver powder having a scale-like shape. The average particle size of the aggregated silver powder and the flaky silver powder is not limited to a specific value. For example, as the aggregated silver powder, those having an average particle size in the range of 4 µm to 8 µm are preferable, and as the flaky silver powder, those having an average particle size in the range of 5 µm to 15 µm are preferable. The total amount of the aggregated silver powder and the flaky silver powder to be compounded can be appropriately set in the range where the electrical conductivity can be imparted, and is, for example, preferably in the range of 50 parts by weight to 500 parts by weight, particularly preferably in the range of 100 parts by weight to 300 parts by weight, based on 100 parts by weight of the liquid silicone rubber. As for the ratio between the content of the aggregated silver powder and the content of flaky silver powder, it is preferable to make the content of the aggregated silver powder and the content of the flaky silver powder equivalent.

Note that the conductive rubber forming the electrode members 3 may further contain other components in addition to the components mentioned above in a range where the effects of the present invention are not compromised. As other components, compounding agents commonly used in the rubber industry, for example, a reinforcing agent, a filler such as dry silica, an anti-aging agent, a processing aid, and a plasticizer can be appropriately compounded.

As mentioned above, the electrode members 3, which are members formed by curing the silicone rubber, have flexibility and resilience, have satisfactory adhesion on the body of a subject, have a soft skin feel, are unlikely to cause discomfort even when allowed to adhere for a long period, and thus can maintain a stable contact with the subject's body.

The electrode members 3 are molded by curing the silicone rubber as a binder including metal particles compounded. On the surface of the intermediate product 40 of each electrode member 3 molded, the molded surface layer 42, which is a layer containing a small amount of metal particles, is formed (see Fig. 4). The thickness of this molded surface layer 42 to be formed is presumed to be approximately 10 µm to 100 µm. Contact impedance between the skin of a subject and the electrode members 3 is defined not by an apparent contact area but by an effective contact area between metal particles responsible for an electrical contact and the skin. For this reason, when the electrode members 3 contact the skin via the molded surface layer 42 after molding, the contact impedance increases, noise to be mixed in biosignals to be detected increases, or obtaining biosignals per se may not be possible.

In contrast, as shown in Fig. 3, each electrode member 3 has the exposed face 32 formed by removal of the molded surface layer 42 at the tip end 31 which contacts the subject, and thus, the electrode members 3 will not contact the subject via the molded surface layer 42. For this reason, it is possible to reduce the contact impedance of the electrode members 3, noise mixed in biosignals to be detected will not increase, and a state in which obtaining biosignals per se is not possible does not occur.

Next, a method for producing the bioelectrode 1 having the configuration mentioned above will be described. The method for producing the bioelectrode 1 includes an electrode member molding step and an exposed face forming step. The electrode member molding step is a step of molding the electrode members 3 by stirring a conductive rubber containing a silicone rubber and metal particles and molding this conductive rubber into a shape projecting from the support member 2, and the exposed face forming step is a step of forming the exposed face 32, which is a face at which the interior of the molded surface layer 42 is exposed, by removing the molded surface layer 42, which is a portion of the surface, at the tip end 31 of each electrode member 3 as the intermediate product 40 molded by the electrode member molding step. Hereinafter, the method for producing the bioelectrode 1 will be specifically described.

The bioelectrode 1, as mentioned above, is integrally molded from the same conductive rubber. In a mold, the conductive rubber is cured, and the support member 2 and electrode members 3 are integrally molded. In other words, the support member 2 and electrode members 3 are integrally molded in the electrode member molding step. However, in an as-molded state in the electrode member molding step, no exposed face 32 is formed in each electrode member 3, and each electrode member 3 is molded in the form of the intermediate product 40 having a tip end part 41 extending beyond the tip end 31 (see Fig. 4). As mentioned above, in the as-molded state from the conductive rubber, the molded surface layer 42, which is a layer containing a small amount of metal particles, is formed on the surface of the molded member. As shown in Fig. 4, the molded surface layer 42 is formed entirely also on the surface of the intermediate product 40 including the tip end part 41 of the electrode member 3.

Next, in the exposed face forming step, the tip end part 41 of the electrode member 3 of the intermediate product 40 is removed, the tip end 31 is formed in the electrode member 3, and the exposed face 32 is formed on the tip end 31 (see Fig. 3). This causes the molded surface layer 42 to be removed in the tip end 31 to thereby expose a portion of the conductive rubber inside the molded surface layer 42, the portion contains a large amount of metal particles. In this manner, the electrode members 3 are formed to thereby complete the bioelectrode 1. Removal of the molded surface layer 42 at the tip end portion 41 of the intermediate product 40 can be conducted by various methods and is conducted by cutting and polishing, for example. An example of cutting is cutting with a cutting tool such as a cutter, and an example of polishing is surface polishing. In each electrode member 3 of the bioelectrode 1 completed, the tip end portion 31 has the exposed face 32, and the exposed face 32 exposes a portion of the conductive rubber inside the molded surface layer 42 which has been removed. At the exposed face 32, more metal particles are exposed than those at the molded surface layer 42. For this reason, it is possible to enlarge the effective contact area, which is the contact area between a subject to be contacted and metal particles at the tip end 31 of each electrode member 3, and the tip end 31 of each electrode member 3 can reduce the contact impedance. The exposed face 32 is preferably one that exposes a large amount of metal particles. In the exposed face forming step, a method of removing the molded surface layer 42 such that metal particles are not removed from the exposed face 32 is preferred to a method of removing the molded surface layer 42 such that metal particles are removed from the exposed face 32. For this reason, as the method of removing the molded surface layer 42 in the exposed face forming step, cutting with a sharp edge tool is preferable.

As mentioned above, in the bioelectrode 1, the electrode members 3 are formed of a conductive rubber, therefore the bioelectrode 1 has satisfactory elasticity so as not to cause discomfort to a subject, and additionally can uniformly adhere to a target site of a subject. For this reason, a reinforcing member, such as a core material for imparting elasticity to the electrode members 3, is not required. It is also possible to form the electrode members 3 only from a conductive rubber, therefore the structure of the electrode members 3 is not complicated, and thus the production can be facilitated. The electrode members 3 have the exposed faces 32 formed by removal of the molded surface layers 42, therefore the bioelectrode 1 does not require use of a conductive gel, electrolyte solution, or the like, are easily used, and additionally cause no discomfort to a subject. It is also possible to reduce the contact impedance by means of the exposed face 32.

As mentioned above, the bioelectrode 1 according to the first embodiment of the present invention has a structure not complicated, has satisfactory elasticity, and can reduce the contact impedance.

Next, an evaluation test for the contact impedance of the bioelectrode 1 according to the first embodiment of the present invention will be described. The present inventors produced the above-described bioelectrodes 1 according to the first embodiment of the present invention (Examples 1 and 2) and conducted a contact impedance evaluation test on the bioelectrodes 1. The present inventors also produced a bioelectrode as a comparative example (Comparative Example 1) and conducted the same contact impedance evaluation test on Comparative Example 1. Comparative Example 1 is a bioelectrode including electrode members as intermediate products 40 as shown in Fig. 4 instead of the electrode members 3 (Fig. 3) in the bioelectrode 1. Comparative Example 1 has the tip end parts 41, the exposed faces 32 are not formed, and Comparative Example 1 has a molded surface layer 42 at the tip end part 41.

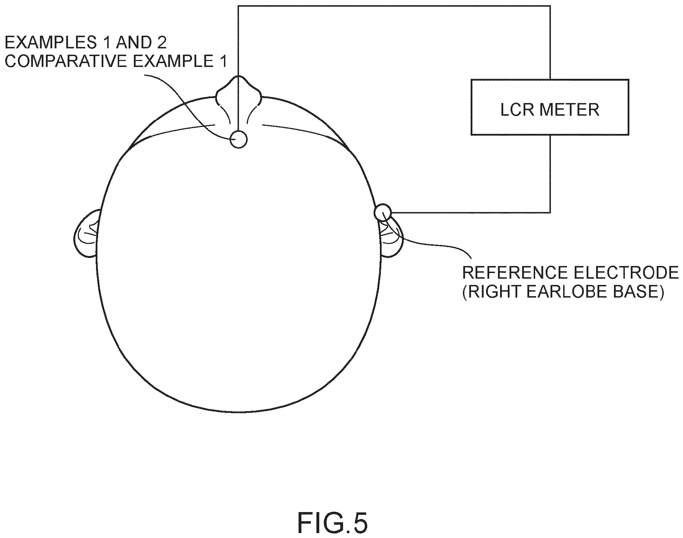

The contact impedance evaluation test was conducted by measuring the contact impedance of each of Examples 1 and 2 and Comparative Example 1 by using an LCR meter as shown in Fig. 5. Specifically, the reference electrode of the LCR meter was fixed to the base of the right earlobe of a subject with an electroencephalographic paste and connected in a state where the contact impedance was sufficiently reduced. Each of Examples 1 and 2 and Comparative Example 1 as an induction electrode was pressed onto the scalp of the subject at a pressing load of 2 N to measure the contact impedance of each of Example 1 and 2 and Comparative Example 1. As measurement value of the contact impedance, value at one minute after the contact of each of the bioelectrodes of Examples 1 and 2 and Comparative Example 1 with the scalp was employed. Note that the following LCR meter was used as the LCR meter and the following electroencephalographic paste was used as the electroencephalographic paste to be used for the reference electrode.
- LCR meter: ZM2371 manufactured by NF Corporation
- Electroencephalographic paste: Ten 20 manufactured by WEAVER and

### COMPANY

As for Examples 1 and 2 and Comparative Example 1, a conductive rubber of components compounded shown in the following Table 1 was centrifuged. The centrifuged rubber was injected into a mold and cross-linked under cross-linking conditions as shown in Table 2. The cross-linked rubber was subjected to a salt water treatment in an autoclave under salt water treatment conditions shown in Table 3 to thereby produce each of the intermediate products 40 of Examples 1 and 2 and the bioelectrodes of Comparative Example 1. Then, for the intermediate products 40 of Examples 1 and 2, the molded surface layer 42 of the tip end part 41 in each electrode member 3 as the intermediate product 40 was removed by the method of removal treatment on molded surface layer shown in the following Table 4 to form the tip end 31 having the exposed face 32 in each electrode member 3, and thus, bioelectrodes of Examples 1 and 2 were each produced. Removal of the molded surface layer 42 in each electrode member 3 as the intermediate product 40 of each of Examples 1 and 2 was conducted by removing the tip end part 41 in a width of approximately 1 mm by each of the method of removal treatment on molded surface layer.

**[Table 1]**

| | Product used (material used) | Amount compounded |
|---|---|---|
| Room temperature-curable liquid silicone rubber | KE-106 manufactured by Shin-Etsu Chemical Co., Ltd. | 100 parts by weight |
| Curing agent | CAT-RG manufactured by Shin-Etsu Chemical Co., Ltd. | 10 parts by weight |
| Silver particles | G-35 manufactured by DOWA HOLDINGS Co., Ltd | 165 parts by weight |
| | FA-2-3 manufactured by DOWA HOLDINGS Co., Ltd | 165 parts by weight |
| Dispersant | KF-6106 manufactured by Shin-Etsu Chemical Co., Ltd. | 10 parts by weight |
| | KF-6015 manufactured by Shin-Etsu Chemical Co., Ltd. | 10 parts by weight |

**[Table 2]**

| | Example 1 | Example 2 | Comparative Example 1 |
|---|---|---|---|
| Primary cross-linking | 150°C, 3 minutes | | |
| Secondary cross-linking | 150°C, 30 minutes | | |

**[Table 3]**

| | Example 1 | Example 2 | Comparative Example 1 |
|---|---|---|---|
| Salt water concentration | 10% | | |
| Immersion time | 1 hour | | |
| Salt water temperature | 121°C | | |
| Pressure | 0.1MPaG | | |

**[Table 4]**

| | Example 1 | Example 2 | Comparative Example 1 |
|---|---|---|---|
| Molded surface layer removal treatment | Buff polishing | Cut with cutter | None |

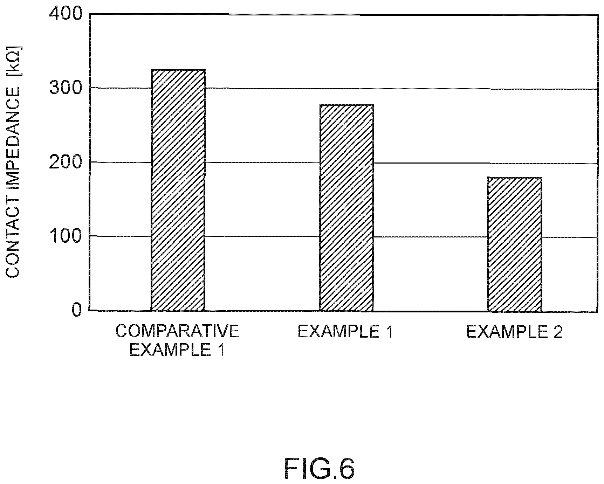

The test results of the present contact impedance evaluation test are shown in Fig. 6. In the present evaluation test, with respect to each of Examples 1 and 2 and Comparative Example 1, the contact impedance was measured three times as mentioned above. The test results are average values of the three measurements. As shown in Fig. 6, the measurement result of the contact impedance of Example 1 was 279 kΩ, the measurement result of the contact impedance of Example 2 was 181 kΩ, and the measurement result of the contact impedance of Comparative Example 1 was 326 kΩ. Accordingly, it can be seen that Examples 1 and 2, in which the exposed face 32, at which the molded surface layer of the tip end part of the electrode member is removed, contacts the scalp of the subject, can reduce the contact impedance more than Comparative Example 1, in which the molded surface layer of the tip end part of each electrode member contacts the scalp of the subject. It also can be seen that Example 2 can reduce the contact impedance more than Example 1 and that the contact impedance will vary depending on methods of removing the molded surface layer 42 of the tip end part 41 in each electrode member 3 as the intermediate product 40. In Example 1, in which the molded surface layer 42 has been removed by buff polishing, some of the silver particles might have been removed from the exposed face 32. It can be seen that Example 2, in which the molded surface layer 42 has been cut-removed with a cutter and silver particles may be unlikely to or may not be removed from the exposed face 32, can reduce the contact impedance more than Example 1.

Hereinafter, a second embodiment of the present invention will be described with reference to the drawings.

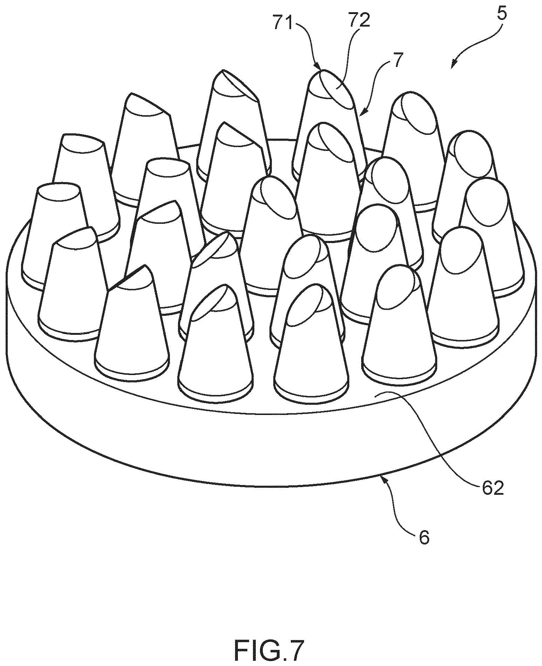

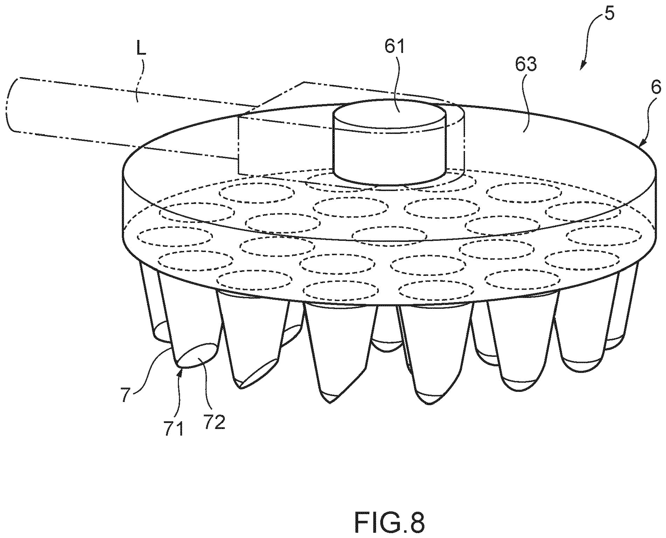

Fig. 7 is a perspective view schematically illustrating a configuration of a bioelectrode according to the second embodiment of the present invention, and Fig. 8 is a perspective view from another direction schematically illustrating the configuration of the bioelectrode illustrated in Fig. 7. As shown in Fig. 7, a bioelectrode 5 includes a support member 6, which is a conductive member, and at least one electrode member 7, which is a member projecting from the support member 6. At least the electrode member 7 is molded from a conductive rubber containing a silicone rubber and metal particles, and the electrode member 7 has an exposed face 72, which is a face formed by removal of a molded surface layer at a tip end portion 71. The exposed face 72 is formed inclinedly with respect to a support face 62 that supports the electrode member 7 in the support member 6, and the tip end portion 71 of the electrode member 7 is pointed.

The tip end portion 71 of the electrode member 7 contacts a body of a subject, and the bioelectrode 5 makes biosignals of the subject detectable via the electrode member 7. The bioelectrode 5 is, for example, a bioelectrode for detecting brainwaves, which contacts a head of a subject to detect brainwaves. The bioelectrode 5 is not limited to these for detecting brainwaves, and is applicable to other devices for detecting biosignals such as wearable information devices. Hereinafter, the configuration of the bioelectrode 5 will be specifically described.

The support member 6 supports the electrode member 7 and is formed of a conductive material so as to be electrically connected to the electrode member 7. In the bioelectrode 5 according to the present embodiment, the support member 6 and the electrode member 7 are integrally molded, and the support member 6 is also formed of the same conductive rubber as that for electrode member 7. The shape of the support member 6 is, as mentioned below, required to be a shape capable of supporting the electrode member 7 in a state where the electrode member 7 is projecting, and is not to be limited to a specific shape. The support member 6 has a disk shape or substantially disk shape, as shown in Fig. 7, for example.

The support member 6 is provided with a terminal 61, which is to be electrically connected to a measurement apparatus, not shown, for receiving biosignals detected by the bioelectrode 5 and processing, analyzing, displaying or the like the biosignals received. The terminal 61 is, for example, as shown in Fig. 8, is connected to a connecting cable L that enables the measurement apparatus to be electrically connected to external devices. The terminal 61 is provided, for example, on a face (terminal side face 63) opposite to the face (support face 62) on which the electrode member 7 is supported, and has a shape to which the connecting cable L can be connected. The terminal 61 is, for example, a projecting portion that projects from the terminal side face 63.

In the support member 6, the terminal 61 is integrally formed with other portions by the same conductive rubber, but the terminal may be one formed as a separate body from the other portions by a different material. In such a case, the terminal 61 can be formed of a material suitable for electrical connection to the connecting cable L. For example, the terminal 61 may be produced from a metal, and may be adhesively provided on the terminal side face 63 of the support member 6 by using a conductive adhesive or may be provided by being embedded more internally than the terminal side face 63 such that a portion of the terminal 61 projects from the terminal side face 63.

A plurality of the electrode members 7 is provided in the bioelectrode 5 as shown in Fig. 7, projecting from the support face 62 of the support member 6 in the same or the substantially same direction. The electrode members 7 project from the support member 6, for example, like a brush, as shown in Fig. 7. The shape of the electrode members 7, as shown in Fig. 7, is, for example, a conical or substantially conical shape, which is a shape having a portion tapering towards the tip end portion 71. The electrode members 7 may have a shape tapering toward the tip end portion 71 entirely, and is required to have a shape having the tip end portion 71 and projecting from the support member 6.

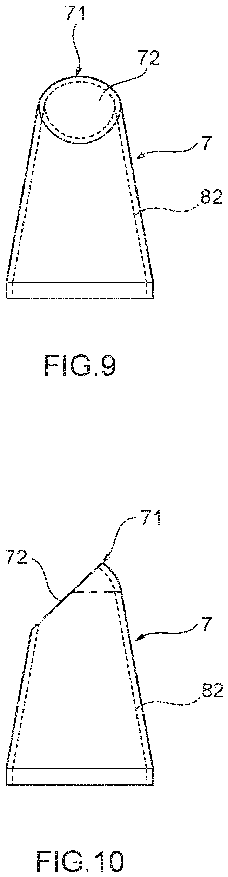

Fig. 9 is a front view of the electrode member 7 of the bioelectrode 5 according to the second embodiment of present invention, and Fig. 10 is a side view of the electrode member 7 of the bioelectrode 5. The tip end part of the tip end portion 71 of the electrode member 7 is pointed. In other words, the shape of the tip end of the tip end portion 71 of each electrode member 7 is a pointed shape in which the electrode member 7 becomes sharper from the support face 62 of the support member 6 toward the tip end side of the electrode member 7. The pointed shape of the tip end portion 71 of the electrode member 7 may be a curvilinearly or substantially curvilinearly pointed shape or may be a linearly pointed shape.

With such a pointed shape, the bioelectrode 5 can reduce contact impedance between a scalp of a subject and the electrode members 7 because the tip end of the pointed tip end portion 71 of each electrode members 7 pushes hair of the subject aside to easily approach the scalp. Also, the electrode members 7 are molded from a flexible conductive rubber. Thus, even when the tip end of the tip end portion 71 is made to have a pointed shape and to contact the scalp, it is possible to cause no pain and make discomfort unlikely to occur.

Additionally, the tip end portion 71 of each electrode member 7 has an exposed face 72, which is a face formed by removal of a molded surface layer, and the exposed face 72 is formed inclinedly with respect to the support face 62 that supports the electrode members 7 in the support member 6. The exposed face 72 is a face diagonally facing with respect toa direction in which the electrode members 7 extend, is a flat face or substantially flat face, and a face diagonally intersecting with the direction in which the electrode members 7 extend.

Each exposed face 72 is elliptical or substantially elliptical, and the exposed faces 72 have the same or the substantially same shape as each other. The exposed faces 72 of the electrode members 7, which are provided in a manner such that the electrode members 7 are concentrically or substantially concentrically arranged at equiangular or substantially equiangular intervals with respect to the center or substantial center of the support member 6, have the same or substantially same inclination with respect to the support face 62 as each other, and are formed along the circumferential direction while facing the same or substantially same direction as each other. Note that the inclination of each exposed face 72 is not particularly limited as long as the tip end of each tip end portion 71 can push hair of a subject aside to thereby allow each tip end portion 71 to approach a scalp.

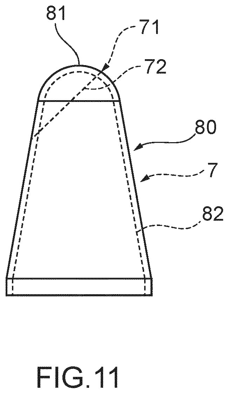

The exposed face 72 may be a curved face or may be a face having a curved face and a flat face. The exposed face 72 is, as mentioned below, a face formed by removing a molded surface layer 82 of a tip end part 81 from an intermediate product 80 of each electrode member 7 molded from a conductive rubber (see Fig. 11), and a cut face or polished face formed respectively by cutting or polishing tip end part 81 of the intermediate product 80 of each electrode member 7.

The conductive rubber forming the electrode members 7 is a rubber containing a silicone rubber and metal particles, as mentioned above. The silicone rubber is, for example, a room temperature-curable liquid silicone rubber, and the metal particles are, for example, silver particles. The metal particles may be any metal particles as long as being a metal-based material having electrical conductivity. The metal particles may be, for example, particles made of a carbon-based material such as carbon black or carbon nanotubes.

As silver particles in the conductive rubber, silver particles in the form of particles containing an aggregated silver powder and a flaky silver powder can be used. The average particle size of the aggregated silver powder and the flaky silver powder is not limited to a specific value. For example, as the aggregated silver powder, those having an average particle size in the range of 4 µm to 8 µm are preferable, and as the flaky silver powder, those having an average particle size in the range of 5 µm to 15 µm are preferable. The total amount of the aggregated silver powder and the flaky silver powder to be compounded can be appropriately set in the range where the electrical conductivity can be imparted, and is, for example, preferably in the range of 50 parts by weight to 500 parts by weight, particularly preferably in the range of 100 parts by weight to 300 parts by weight, based on 100 parts by weight of the liquid silicone rubber. As for the ratio between the content of the aggregated silver powder and the content of flaky silver powder, it is preferable to make the content of the aggregated silver powder and the content of the flaky silver powder equivalent.

Note that the conductive rubber forming the electrode members 7 may further contain other components in addition to the components mentioned above in a range where the effects of the present invention are not compromised. As other components, compounding agents commonly used in the rubber industry, for example, a reinforcing agent, a filler such as dry silica, an anti-aging agent, a processing aid, and a plasticizer can be appropriately compounded.

As mentioned above, the electrode members 7, which are members formed by curing the silicone rubber, have flexibility and resilience, have satisfactory adhesion on the body of a subject, have a soft skin feel, are unlikely to cause discomfort even when allowed to adhere for a long period, and thus can maintain a stable contact with the subject's body.

Fig. 11 is a side view of the electrode member 7 in the intermediate product 80 molded in an electrode member molding step of a method for producing a bioelectrode 5 according to the second embodiment of present invention. The electrode member 7 is molded by curing the silicone rubber as a binder including metal particles compounded. On the surface of the intermediate product 80 of the electrode member 7 molded, a molded surface layer 82, which is a layer containing a small amount of metal particles, is formed. Contact impedance between a skin of a subject and the electrode members 7 is defined not by an apparent contact area but by an effective contact area between metal particles responsible for an electrical contact and a skin. For this reason, when the electrode members 7 contact the skin via the molded surface layer 82 after molding, the contact impedance increases, noise to be mixed in biosignals to be detected increases, or obtaining biosignals per se may not be possible.

In contrast, as shown in Figs. 9 and 10, each electrode member 7 has the exposed face 72 formed by removal of the molded surface layer 82 at the tip end portion 71 which contacts a subject, and thus, the electrode members 7 will not contact a subject via the molded surface layer 82. For this reason, it is possible to reduce the contact impedance of the electrode members 7, noise mixed in biosignals to be detected will not increase, and a state in which obtaining biosignals per se is not possible does not occur.

Next, a method for producing a bioelectrode 5 having the configuration mentioned above will be described. The method for producing the bioelectrode 5 includes an electrode member molding step and an exposed face forming step. The electrode member molding step is a step of molding the electrode members 7 by stirring a conductive rubber containing a silicone rubber and metal particles and molding this conductive rubber into a shape projecting from the support member 6, and the exposed face forming step is a step of forming the exposed face 72, which is a face at which the interior of the molded surface layer 82 is exposed, by removing the molded surface layer 82, which is a portion of the surface, at the tip end portion 71 of each electrode member 7 as the intermediate product 80 molded by the electrode member molding step. In the exposed face forming step, the exposed face 72 is formed inclinedly with respect to the support face 62 that supports the electrode members 7 in the support member 6, and the tip end portion 71 is formed pointed. Hereinafter, the method for producing the bioelectrode 5 will be specifically described.

The bioelectrode 5, as mentioned above, is integrally molded from the same conductive rubber. In a mold, the conductive rubber is cured, and the support member 6 and electrode members 7 are integrally molded. In other words, the support member 6 and electrode members 7 are integrally molded in the electrode member molding step. However, in an as-molded state in the electrode member molding step, no exposed face 72 is formed in each electrode member 7, and each electrode member 7 is molded in the form of the intermediate product 80 having a tip end part 81 extending beyond the exposed face 72 (see Fig. 11). As mentioned above, in the as-molded state from the conductive rubber, the molded surface layer 82, which is a layer containing a small amount of metal particles, is formed on the surface of the molded member. As shown in Fig. 11, the molded surface layer 82 is formed entirely also on the surface of the intermediate product 80 including the tip end part 81 of the electrode member 7.

Next, in the exposed face forming step, the tip end part 81 of the electrode member 7 of the intermediate product 80 is diagonally removed inclinedly with respect to the support face 62, and the exposed face 72 is formed in the tip end portion 71 (see Figs. 9 and 10). This causes the molded surface layer 82 to be removed in the tip end portion 71 to thereby expose a portion of the conductive rubber inside the molded surface layer 82, the portion contains metal particles in a large amount. In this manner, the electrode members 7 are formed to thereby complete the bioelectrode 5.

Removal of the molded surface layer 82 at the tip end part 81 of the intermediate product 80 can be conducted by various methods and is conducted by cutting and polishing, for example. An example of cutting is cutting with a cutting tool such as a cutter, and an example of polishing is surface polishing. In each electrode member 7 of the bioelectrode 5 completed, the tip end portion 71 has the exposed face 72, and the exposed face 72 exposes a portion of the conductive rubber inside the molded surface layer 82 which has been removed. At the exposed face 72, more metal particles are exposed than those at the molded surface layer 82. For this reason, it is possible to enlarge the effective contact area, which is the contact area between a subject to be contacted and metal particles at the tip end portion 71 of each electrode member 7, and the tip end portion 71 of each electrode member 7 can reduce the contact impedance.

The exposed face 72 is preferably one that exposes a large amount of metal particles. In the exposed face forming step, a method of removing the molded surface layer 82 such that metal particles are not removed from the exposed face 72 is preferred to a method of removing the molded surface layer 82 such that metal particles are removed from the exposed face 72. For this reason, as the method of removing the molded surface layer 82 in the exposed face forming step, cutting with a sharp edge tool is preferable.

As mentioned above, in the bioelectrode 5, the electrode members 7 are formed of a conductive rubber, therefore the bioelectrode 5 has satisfactory elasticity so as not to cause discomfort to a subject, and additionally can uniformly adhere to a target site of a subject. For this reason, a reinforcing member, such as a core material for imparting elasticity to the electrode members 7, is not required. It is also possible to form the electrode members 7 only from a conductive rubber therefore the structure of the electrode members 7 is not complicated, and thus the production can be facilitated.

The electrode members 7 have the exposed faces 72 formed by removal of the molded surface layer 82, therefore the bioelectrode 5 does not require use of a conductive gel, electrolyte solution, or the like, are easily used, and additionally cause no discomfort to a subject. It is also possible to reduce the contact impedance by means of the exposed face 72. Additionally, the tip end part of the tip end portion 71 of each electrode member 7 is pointed. Thus, it is possible to reduce the contact impedance between a scalp and the electrode members 7 because the tip end of the pointed tip end portion 71 of each electrode member 7 pushes hair of a subject aside to easily approach the scalp. Also, the electrode members 7 are molded from a flexible conductive rubber. Thus, even when the tip end of the tip end portion 71 is made to have a pointed shape and to contact a scalp, it is possible to cause no pain and make discomfort unlikely to occur.

As mentioned above, the bioelectrode 5 according to the second embodiment of the present invention has a structure not complicated, has satisfactory elasticity, and can reduce the contact impedance.

Next, an evaluation test for the contact impedance of the bioelectrode 5 according to the second embodiment of the present invention will be described. The present inventors produced the above-described bioelectrode 5 according to the second embodiment of the present invention (Example 3), and conducted a contact impedance evaluation test on the bioelectrode 5. The present inventors also produced a bioelectrode as a Comparative Example (Comparative Example 2) and conducted the same contact impedance evaluation test on Comparative Example 2.

Comparative Example 2 is a bioelectrode including electrode members as intermediate products 80 as shown in Fig. 11 instead of electrode members 7 (Figs. 9 and 10) in the bioelectrode 5. Comparative Example 2 has the tip end parts 81, the exposed faces 72 are not formed, and Comparative Example 2 has a molded surface layer 82 at the tip end portion 71.

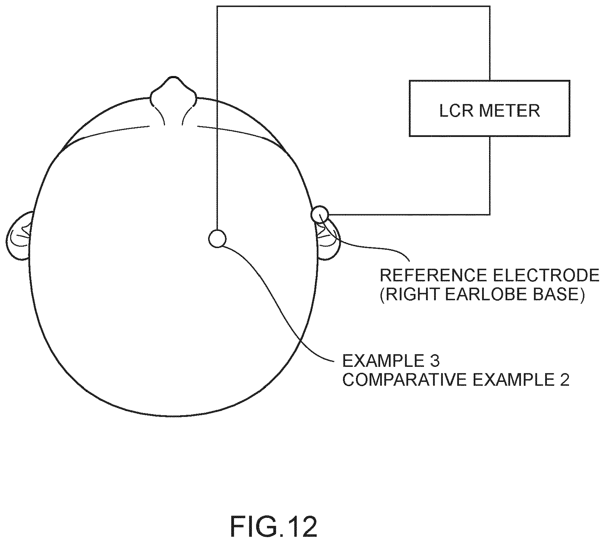

The contact impedance evaluation test was conducted by measuring the contact impedance of each of Example 3 and Comparative Example 2 by using an LCR meter as shown in Fig. 12. Specifically, the reference electrode of the LCR meter was fixed to a base of a right earlobe of a subject with an electroencephalographic paste and connected in a state where the contact impedance was sufficiently reduced. Each of Example 3 and Comparative Example 2 as an induction electrode was pressed onto the scalp of the subject at a pressing load of 2 N to measure the contact impedance of each of Example 3 and Comparative Example 2.

As measurement value of the contact impedance, value at one minute after the contact of each of Example 3 and Comparative Example 2 with the scalp was employed. Note that the following LCR meter was used as the LCR meter and the following electroencephalographic paste was used as the electroencephalographic paste to be used for the reference electrode.
- LCR meter: ZM2371 manufactured by NF Corporation
- Electroencephalographic paste: Ten 20 manufactured by WEAVER and COMPANY

As for Example 3 and Comparative Example 2, a conductive rubber of components compounded shown in the following Table 5 was centrifuged. The centrifuged rubber was injected into a mold and cross-linked under cross-linking conditions as shown in Table 6. The cross-linked rubber was subjected to a salt water treatment in an autoclave under salt water treatment conditions shown in Table 7 to thereby produce each of the intermediate products 80 of Example 3 and Comparative Example 2.

Then, for the intermediate product 80 of Example 3, the molded surface layer 82 of the tip end part 81 in each electrode member 7 as the intermediate product 80 is removed diagonally with respect to the support face 62 by the method of removal treatment on molded surface layer shown in the following Table 8 to form the tip end portion 71 having the exposed face 72 in each electrode member 7, and thus the bioelectrode of Example 3 was produced.

**[Table 5]**

| | Product used (material used) | Amount compounded |
|---|---|---|
| Room temperature-curable liquid silicone rubber | KE-106 manufactured by Shin-Etsu Chemical Co., Ltd. | 100 parts by weight |
| Curing agent | CAT-RG manufactured by Shin-Etsu Chemical Co., Ltd. | 10 parts by weight |
| Silver particles | G-35 manufactured by DOWA HOLDINGS Co., Ltd | 165 parts by weight |
| | FA-2-3 manufactured by DOWA HOLDINGS Co., Ltd | 165 parts by weight |
| Dispersant | KF-6106 manufactured by Shin-Etsu Chemical Co., Ltd. | 10 parts by weight |
| | KF-6015 manufactured by Shin-Etsu Chemical Co., Ltd. | 10 parts by weight |

**[Table 6]**

| | Example 3 | Comparative Example 2 |
|---|---|---|
| Primary cross-linking | 150°C, 3 minutes | |
| Secondary cross-linking | 150°C, 30 minutes | |

**[Table 7]**

| | Example 3 | Comparative Example 2 |
|---|---|---|
| Salt water concentration | 10% | |
| Immersion time | 1 hour | |
| Salt water temperature | 121°C | |
| Pressure | 0.1MPaG | |

**[Table 8]**

| | Example 3 | Comparative Example 2 |
|---|---|---|
| Molded surface layer removal treatment | Cut diagonally with cutter | None |

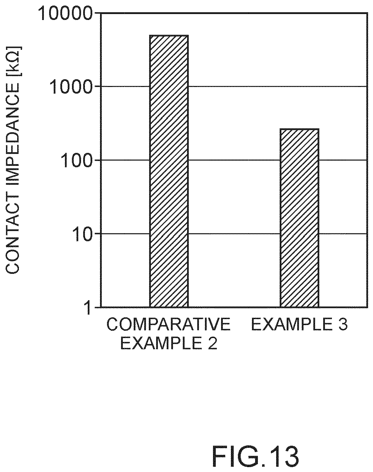

The test results of the present contact impedance evaluation test are shown in Fig. 13. In the present evaluation test, with respect to each of Example 3 and Comparative Example 2, the contact impedance was measured three times as mentioned above. The test results are average values of the three measurements. As shown in Fig. 13, the measurement result of the contact impedance of Example 3 was 264 kΩ, and the measurement result of the contact impedance of Comparative Example 2 was 5300 kΩ.

Accordingly, it can be seen that Example 3, in which the exposed face 72, at which the molded surface layer of the tip end part of each electrode member is removed, contacts the scalp of the subject, can reduce the contact impedance more than Comparative Example 2, in which the molded surface layer of the tip end part of each electrode member contacts the scalp of the subject.

Although the embodiments of the present invention has been described hereinabove, the present invention is not limited to the embodiments of the present invention described above and includes any aspects included in the concept and claims of the present invention. The configurations each may be appropriately and selectively combined to solve or provide at least part of the above-described problems or effects. For example, the shape, material, arrangement, size, and the like of each component in the embodiments described above may be appropriately changed in accordance with specific use aspects of the present invention.

For example, the shape of the support member 2, 6 is not limited to the shape mentioned above and may be a different shape. Similarly, the shape of the electrode member 3, 7 is not limited to the shape mentioned above and may be a different shape.

The support member 2, 6 and the electrode member 3, 7 may be separate bodies from each other. The electrode member 3, 7, after formed by the electrode member molding step and the exposed face forming step mentioned above, may be attached and fixed to the support member 2, 6 formed as separate bodies to thereby form the bioelectrode 1, 5. Fixing of the electrode member 3, 7 to the support member 2, 6 may be achieved with a conductive adhesive or may be achieved with engagement such as fitting. For example, a recessed part or protruding part is formed at the bottom of the electrode member 3, 7, and the corresponding protruding part or recessed part is formed in the support member 2, 6. The recessed part and the protruding part are engaged with each other to thereby enable the electrode member 3, 7 to be fixed to the support member 2, 6. The electrode member 3, 7 may be detachably fixed to the support member 2, 6.

When the support member 2, 6 and the electrode member 3, 7 are separate bodies, both the support member 2, and the electrode member 3, 7 may be formed with the same material using the conductive rubber mentioned above or may be formed with different materials. When the support member 2, 6 is formed with a material different from that of the electrode member 3, 7, the support member 2, 6 can be formed with a material having electrical conductivity different from the conductive rubber. As the material having electrical conductivity for the support member 2, 6, a material having electrical conductivity suitable for supporting the electrode member 3, 7 is preferable. For example, it is possible to use a metal, such as stainless, copper, or aluminum, having a strength enough to stably support the electrode member 3, 7. The material of the support member 2, 6 is not limited thereto, and materials having electrical conductivity can be used.

### List of Reference Signs

1, 5 bioelectrode, 2, 6 support member, 21, 61 terminal, 22, 62 support face, 23, 63 terminal side face, 3, 7 electrode member, 31, 71 tip end, 32, 72 exposed face, 40, 80 intermediate product, 41, 81 tip end part, 42, 82 molded surface layer, L connecting cable

## Claims

1. A bioelectrode **characterized by** comprising:
a support member, which is a conductive member, and
at least one electrode member, which is a member projecting from the support member,
wherein at least the electrode member is molded from a conductive rubber containing a silicone rubber and metal particles, and
the electrode member has an exposed face, which is a face formed by removal of a molded surface layer at a tip end portion.

2. The bioelectrode according to claim 1, **characterized in that**
the exposed face is formed inclinedly with respect to a support face that supports the electrode member in the support member, and
the tip end portion is pointed.

3. The bioelectrode according to claim 2, **characterized in that** an inclination of each exposed face is the same with respect to the support face.

4. The bioelectrode according to any one of claims 1 to 3, **characterized in that** the exposed face is a cut face.

5. The bioelectrode according to any one of claims 1 to 3, **characterized in that** the exposed face is a polished face.

6. The bioelectrode according to any one of claims 1 to 5, **characterized in that** the exposed face is a flat face.

7. The bioelectrode according to any one of claims 1 to 6, **characterized in that** the silicone rubber is a room temperature-curable liquid silicone rubber.

8. The bioelectrode according to any one of claims 1 to 7, **characterized in that** the metal particles are silver particles.

9. A method for producing a bioelectrode comprising a support member, which is a conductive member, and at least one electrode member, which is a member projecting from the support member, **characterized by** comprising:
an electrode member molding step of molding the electrode member by stirring a conductive rubber containing a silicone rubber and metal particles and molding the conductive rubber into a shape projecting from the support member, and
an exposed face forming step of forming an exposed face, which is a face at which an interior of a molded surface layer is exposed, by removing the molded surface layer, which is a portion of the surface, at a tip end portion of the electrode member molded by the electrode member molding step.

10. The method for producing a bioelectrode according to claim 9, **characterized in that**, in the exposed face forming step, the exposed face is formed inclinedly with respect to a support face that supports the electrode member in the support member, and the tip end portion is formed in a manner such that the tip end portion is pointed.
